# FASCICULE DE BREVET EUROPEEN

(11) **EP 3 319 584 B1**
(45) Date de publication et mention de la délivrance du brevet: **12.08.2020**
(21) Numéro de dépôt: 16744802.6
(22) Date de dépôt: 05.07.2016
(51) Int. Cl.: A61Q 15/00, A61K 8/42, A61K 8/9789

(54) **UTILISATION DE N-ALKYLAMIDE COMME AGENT ANTI-TRANSPIRANT**
VERWENDUNG VON N-ALKYL-AMID ALS SCHWEISSHEMMENDES MITTEL
USE OF N-ALKYL AMIDE AS AN ANTIPERSPIRANT AGENT

(30) Priorité: 07.07.2015 FR 1556438
(43) Date de publication de la demande: 16.05.2018
(73) Titulaire: Robertet S.A., 06130 Grasse (FR)
(72) Inventeur: PEGARD, Anthony, 06130 Grasse (FR)
(74) Mandataire: Agasse, Stéphane
(86) Numéro de dépôt international: PCT/FR2016/051693
(87) Numéro de publication internationale: WO 2017/006042

(56) Documents cités:
- EP-A2- 0 319 486
- EP-A2- 1 121 927
- JP-A- 2003 292 427

## Description

La transpiration permet au corps de maintenir sa température quand il fait chaud, la sueur assurant le refroidissement de la peau.

Une transpiration importante, voire excessive peut s'avérer gênante.

Lors des phases de transpiration, les glandes sudoripares sécrètent la sueur qui contient essentiellement de l'eau, mais également des minéraux et du lactate.

Chez l'homme, on distingue deux sortes de glandes sudoripares qui diffèrent par leurs fonctions et par la composition de la sueur qu'elles excrètent :
- les glandes sudoripares « eccrines » ; et
- les glandes sudoripares « apocrines ».

Les glandes sudoripares eccrines sont de loin les plus nombreuses et se localisent sur presque tout le corps mais surtout sur la paume des mains, sur la plante des pieds et sur le front. Chacune d'elle est une glande simple, tubuleuse et en spirale, dont l'extrémité, le glomérule, se situe dans l'épaisseur du derme ou dans le tissu sous-cutané.

La sueur sécrétée par les glandes sudoripares eccrines est composée à 99% d'eau et d'électrolytes, notamment du chlorure de sodium, et d'environ 1% de composés organiques, notamment d'acide lactique. Bien que la sueur sécrétée par les glandes sudoripares eccrines ne possède pas en soit d'odeur, elle peut, dans certaines conditions de macération, être à l'origine d'infections bactériennes ou d'irritations.

Les glandes sudoripares apocrines se situent notamment sous les aisselles. Elles sont plus grosses que les glandes eccrines et leur conduit excréteur débouche dans un follicule pileux.

Outre les composants de base identiques à ceux de la sueur excrétée par les glandes eccrines, la sueur excrétée par les glandes apocrines contient également des molécules organiques (lipides et protéines) dont des phéromones qui, une fois transformées par des bactéries cutanées, sont à l'origine des odeurs dites « de transpiration ».

Pour prévenir l'apparition de ces mauvaises odeurs, on utilise notamment des compositions déodorantes plus classiquement désignées par « déodorants ». Les déodorants peuvent agir de diverses manières :
- en masquant les mauvaises odeurs, par exemple grâce à des ingrédients parfumés ;
- en absorbant la sueur et en limitant la diffusion des molécules « odorantes », par exemple grâce à du talc ;
- en limitant la production de sueur, par exemple grâce à la toxine botulique,
- en agissant directement sur les bactéries qui, en métabolisant des constituants de la sueur apocrine, dégagent l'odeur attribuée à la transpiration.

Les compositions visant à réduire la production de sueur et à prévenir l'apparition de mauvaises odeurs comprennent des principes actifs anti-transpirants.

Parmi les principes actifs anti-transpirants disponibles, on trouve des sels d'aluminium, sels de zirconium, et les sels d'aluminium/zirconium, en vue de réduire la production de sueur. Cependant, l'utilisation topique de l'aluminium, du zirconium, et les sels d'aluminium/zirconium semble poser des problèmes de sécurité sanitaire, tels que décrits dans les publications concernant les effets indésirables liés à l'utilisation des anti-transpirants. De plus, l'acidité de l'aluminium, du zirconium, et les sels d'aluminium/zirconium les rend potentiellement irritants pour la peau. Les anti-transpirants à base d'aluminium, de zirconium et d'aluminium/zirconium sont également sensibles aux métaux, tels que le fer, qui peuvent déstabiliser l'anti-transpirant et provoquer la formation de taches sur les tissus. JP 2003 292427 décrit des extraits de plants, par exemple Zanthoxylum piperitum (comprenant des sanshools), comme agents déodorants capables de combattre la formation de mauvaises odeurs associées avec la transpiration.

EP 1 121 927 décrit l'utilisation de spilanthol et de sanshools comme agents produisant une sensation de picotement utilisés dans des compositions anti-transpirantes.

Or, il a maintenant été trouvé que des composés de la famille des N-alkylamides peuvent de façon tout à fait surprenante, limiter la production de sueur.

La présente invention a donc pour objet l'utilisation d'au moins un N-alkylamide tel que défini dans les revendications 1 et 2 comme agent anti transpirant. Les N-alkylamides de l'invention permettent de réduire efficacement la transpiration, et de ce fait l'humidité liée à la transpiration et les mauvaises odeurs, que ce soit en l'absence d'un anti-transpirant à base d'aluminium actif ou en combinaison avec de faibles quantités d'un anti-transpirant à base d'aluminium actif et/ou zirconium.

Les N-alkylamides peuvent être des produits de synthèse ou des extraits naturels de plantes. Parmi les N-alkylamides susceptibles d'être utilisés dans le cadre de l'invention on peut citer: le spilanthol, et les sanshools comprenant notamment l'hydroxysanshool; l'α-sanshool; l'α-hydroxysanshool; le β-hydroxysanshool; le δ-sanshool; le γ-hydroxysanshool; le γ-hydroxyisosanshool; le γ-dehydrosanshool; le γ-sanshool.

Selon un mode de réalisation la composition cosmétique comprend au moins deux N-alkylamides. En effet, l'utilisation d'au moins deux N-alkylamides dans une composition cosmétique selon l'invention permet d'obtenir un effet synergique.

Les deux N-alkylamides sont le spilanthol et des sanshools.

Dans une mise en oeuvre de l'invention, la source de spilanthol est un extrait oléorésine *d'Acmella oleacerae* ou du spilanthol pur et la source de sanshools est un extrait CO₂ supercritique de Zanthoxylum *piperitum.*

A titre d'exemple, un extrait oléorésine *d'Acmella oleacerae* peut comprendre 32% de spilanthol, un extrait CO₂ supercritique de Zanthoxylum *piperitum* peut comprendre par exemple entre 18 et 50% de sanshools.

Selon un autre aspect de l'invention, la quantité de spilanthol dans la composition est comprise entre 0,05% et 10 % en poids, avantageusement 0,05 et 5 % et plus préférentiellement entre 0,05 et 1% et notamment 0, 08 % ou 0,16% en poids de la composition. La quantité en sanshools est comprise entre 0,03% et 25 % en poids, de préférence entre 0,04% et 5 %, et de façon préférentielle, la quantité de sanshools est comprise entre 0,045 et 0,25% ou encore entre 0,09 % et 0,125% en poids de la composition.

La formulation cosmétique peut éventuellement comprendre des composants qui favorisent la pénétration N-alkylamides. Des exemples de promoteurs de pénétration comprennent l'urée, le polyoxyéthylène (par exemple, Brij-30 et laureth-4), 3-hydroxy-3,7, 11 - triméthyl-1 ,6,10-dodecatriene, acides cis-gras (par exemple, l'acide oléique , l'acide palmitoléique), l'acétone, le laurocaprame, le diméthylsulfoxyde, la 2-pyrrolidone, l'alcool oléique, le glycérol-3-stéarate, le propan-2-ol, ester de l'acide myristique d'isopropyle, le cholestérol, le nérolidol , les bisabolols et le propylene glycol, ainsi que ses dérivés.

Dans un autre aspect de l'invention, l'activateur de pénétration de la composition est le nérolidol.

La quantité d'activateur de pénétration est comprise entre 1 et 50% en poids de la composition cosmétique ; de préférence lorsque l'activateur de pénétration est le nérolidol, la quantité est comprise entre 0,05 et 1% et de façon préférentielle de 1% en poids de la composition.

Les N-alkylamides selon l'invention sont soit intégrés directement dans la composition cosmétique soit introduits dans la composition cosmétique sous la forme d'un parfum, ledit parfum comprenant le ou les N-alkylamides et éventuellement l'activateur de pénétration. Dans le cas où les N-alkylamides sont introduits sous la forme d'un parfum, le parfum représente par exemple de 0,5 à 5% en poids de la composition cosmétique, de préférence 1%.

Selon un autre aspect, l'invention a également pour objet un procédé de traitement cosmétique de la transpiration consistant à appliquer, par voie locale, une quantité efficace d'une composition à base d'au moins un N-alkylamide selon la revendication 7. Dans une mise en oeuvre particulière la composition appliquée comprend au moins deux N-alkylamides. selon la revendication 8. Divulguée est également une composition cosmétique comprenant à titre de principe actif au moins du spilanthol pur ou sous forme d'un extrait oléorésine de Jambu *(Acmella oleracea)* et des sanshools sous forme d'extrait CO₂ supercritique de poivre de Sichuan *(Zanthoxylum piperitum).* Cette composition peut également comprendre un composé favorisant la pénétration des principes actifs. Dans une mise en oeuvre particulière de l'invention, l'agent de pénétration est le nérolidol.

Dans une autre mise en oeuvre la composition cosmétique comprend entre 0,05% et 10 %, avantageusement de 0,05 et 5 %, et plus préférentiellement encore entre 0,05 et 1% et notamment 0,08 ou 0,16% de spilanthol en poids de la composition, et/ou entre 0,03 et 25%, avantageusement de 0,04 et 5%, et notamment entre 0,045 et 0,25% ou entre 0,09 % et 0,125% de sanshools en poids de la composition et optionnellement 0,05 et 1%, et de préférence 1 % en poids de la composition de nérolidol.

Les compositions cosmétiques peuvent être formulées sous toute forme galénique appropriée à leur administration. Les compositions selon la présente invention peuvent ainsi être formulées sous forme de crème, gel, lotion, lait, émulsion huile dans eau ou eau dans huile, parfum, solution, onguent, pulvérisateur, huile corporelle, ou bâton.

Les compositions cosmétiques peuvent prendre la forme d'une crème ou d'un gel dans lesquels au moins un N-alkylamide, de préférence deux N-alkylamides, est mélangé aux excipients classiquement utilisés dans le domaine cosmétique.

Les compositions cosmétiques peuvent prendre la forme de gels dans les excipients appropriés tels que les esters de cellulose ou d'autres agents gélifiants, tels que Carbopol, Sepinov (polyacrylate), la gomme guar, etc.

Les compositions cosmétiques peuvent aussi prendre la forme d'une lotion ou d'une solution dans lesquelles au moins un N-alkylamide est sous forme encapsulée. Les microsphères peuvent par exemple être constituées de corps gras, d'agar et d'eau. Les N-alkylamides selon la présente invention peuvent être incorporés dans des vecteurs de type liposomes, glycosphères, cyclodextrines, dans des chylomicrons, des macro-, micro-, nano-particules ainsi que les macro-, micro- et nanocapsules et aussi être absorbés sur des polymères organiques poudreux, les talcs, bentonites et autres supports minéraux.

Ces émulsions jouissent d'une bonne stabilité et peuvent être conservées pendant le temps nécessaire pour l'utilisation à des températures comprises entre 0 et 50°C sans qu'il y ait sédimentation des constituants ou séparation des phases.

Les compositions cosmétiques peuvent aussi contenir des additifs ou des adjuvants usuels en cosmétologie, comme par exemple des agents antimicrobiens ou des parfums mais aussi des lipides d'extraction ou de synthèse, des polymères gélifiants et viscosants, des tensio-actifs et des émulsifiants, des principes actifs hydro- ou liposolubles, des extraits de plantes, des extraits tissulaires, des extraits marins, des actifs de synthèse.

Les compositions cosmétiques peuvent aussi comprendre d'autres principes actifs complémentaires choisis pour leur action. Lorsque les compositions contiennent des principes actifs complémentaires, ceux-ci sont généralement présents dans la composition à une concentration suffisamment élevée pour qu'ils puissent exercer leur activité.

Les compositions cosmétiques sont de préférence utilisées quotidiennement et appliquées une ou plusieurs fois par jour.

Les exemples suivants sont illustrés.

### Exemples

### Exemple1 :

Le matériel utilisé est le suivant :
- patchs de coton de taille 7.5 cm x 7.5 cm

Matières premières utilisées en parfumerie :
- Oleorésine de Jambu *(Acmella oleacerae)* CAS n° 90131-24-1, titrant à 32% de spilanthol,
- Extrait CO2 de poivre de Sichuan *(Zanthoxylum piperitum)* CAS n° 102242-62-6, titrant à 18% d'hydroxy sanshoolalpha et beta,
- 1% de nérolidol CAS n° 7212-44-4

Des patchs de cotons permettant de récupérer la sueur produite au cours d'un effort reproductible sont disposés sous les aisselles.

La transpiration produite au cours d'une heure d'effort est ensuite mesurée par pesage.

La différence entre l'aisselle traitée (droite) et l'aisselle non-traitée (gauche) est mesurée, ainsi que la différence entre aisselle droite traitée avec un placebo et aisselle droite traitée avec la formule active.

L'effort est effectué pendant une heure à une température de 20°C.

Les expériences sont réalisées en triplicats.

### Exemple 2 (pas selon l'invention):

Application sur l'aisselle droite de 5 pulvérisations (170 mg) d'une solution d'alcool à 85% comprenant soit 0.5% de Jambu titrant à 32% de spilanthol (ou 0,16% de spilanthol pur) et 1% de nérolidol soit uniquement de l'alcool à 85% (contrôle), et aucune application sur l'aisselle gauche.

Après l'effort, à t+1h, le poids de sueur recueillie sur les patchs droits est mesuré :
Moyenne Contrôle (moyenne des résultats obtenus pour l'aisselle droite, i.e., sur laquelle est pulvérisée de l'alcool uniquement*): **3.62 g ±0.3 g**
Moyenne Tests (moyenne des résultats obtenus pour l'aisselle droite, i.e., sur laquelle est pulvérisée la solution d'alcool et d'oléorésine de Jambu **): 2.68 g ±0.6 g

### Poids de sueur recueillie sur les patchs gauches :

Moyenne Contrôle (cf. *): 1,73 g
Moyenne Tests (cf. **): 2,41 g ± 0.3g

### Différentiel aisselle traitée (aisselle droite) / aisselle non traitée (aisselle gauche) :

Moyenne Contrôle : 109%
Moyenne des Tests : **13,65** %

### Différentiel aisselle traitée avec produit actif (cf. **)/ aisselle traitée avec placebo (cf. *) :

Moyenne des tests : **-25,7** %

### Exemple 3 :

Application soit de 5 pulvérisations (170 mg) d'une solution d'alcool à 85% comprenant 0.5% d'extrait CO2 supercritique de poivre de Sichuan titrant à 18% de sanshools et 1% de nérolidol uniquement sur l'aisselle droite soit uniquement de l'alcool à 85% (contrôle), et aucune application sur l'aisselle gauche

Après l'effort, à t+1h, le poids de sueur recueillie sur les patchs droits est mesuré :
Moyenne Contrôle (moyenne des résultats obtenus pour l'aisselle droite, i.e., sur laquelle est pulvérisée de l'alcool uniquement*): 2.84 g ±0.3 g
Moyenne Tests (moyenne des résultats obtenus pour l'aisselle droite, i.e., sur laquelle est pulvérisée la solution d'alcool, et d'extrait de poivre de Sichuan**) : 2.25 g ±0 g

### Poids de sueur recueillie sur les patchs gauches :

Moyenne Contrôle : 2,3 g ± 0.2 g
Moyenne Tests : 2,31 g ± 0 g

### Différentiel aisselle traitée / aisselle non traitée :

Moyenne Contrôle : 23,5%
Moyenne des tests : **-2,65**%

### Différentiel aisselle traitée avec produit actif / aisselle traitée avec placebo

Moyenne des tests : **-20,75**%

### Exemple 4

Application soit de 5 pulvérisations (170 mg) d'une solution d'alcool à 85% comprenant le mélange suivant sur l'aisselle droite.
- 0.25% de Jambu *(Acmella oleacerae)* Oleorésine CAS n° 90131-24-1, titrant à 32% de spilanthol (ou 0.08% de spilanthol pur CAS n° 25394-57-4 (fabrication Robertet)), et
- 0.25% d'extrait CO2 de poivre de Sichuan *(Zanthoxylum piperitum)* CAS n° 102242-62-6, titrant à 18% d'hydroxy sanshoolalpha et beta, et
- 1% de nérolidol CAS n° 7212-44-4
soit uniquement de l'alcool à 85% (contrôle), et aucune application sur l'aisselle gauche

Après l'effort, à t+1h, le poids de sueur recueillie sur les patchs droits est mesuré :
Moyenne Contrôle (moyenne des résultats obtenus pour l'aisselle droite, i.e., sur laquelle est pulvérisée de l'alcool uniquement*): 3.79 g ±0.3 g
Moyenne Tests (moyenne des résultats obtenus pour l'aisselle droite, i.e., sur laquelle est pulvérisée la solution d'alcool, d'oléorésine de Jambu et d'extrait de poivre de Sichuan**) : 2.37 g ±0.6 g

### Poids de sueur recueillie sur les patchs gauches :

Moyenne Contrôle *: 3.54 g ± 0.4 g
Moyenne Tests** : 2.56 g ± 0.5 g

### Différentiel aisselle traitée / aisselle non traitée :

Contrôle : 6%
Moyenne des tests : **-7,95**%

### Différentiel aisselle traitée avec produit actif / aisselle traitée avec placebo

Moyenne des tests : **-32,9**%

### Exemple 5

Composition aérosol selon l'invention comprenant :
- Oleorésine de Jambu *(Acmella oleacerae)* CAS n° 90131-24-1, titrant à 30% de spilanthol : 0,3%
- Extrait CO2 de poivre de Sichuan *(Zanthoxylum piperitum)* CAS n° 102242-62-6, titrant à 16% d'hydroxy sanshoolalpha et beta : 0,3%
- Nérolidol CAS n° 7212-44-4 : 1%,
- Dicarylyl Carbonate : 2,6%
- Cyclomethicone : 25.8%
- AEROGAZ 2.5 (PROPANE-BUTANE-2.5 BARS) : 70%

### METHODOLOGIE

La solution aérosol selon l'invention a été utilisée sur 14 volontaires adultes de 18 à 65 ans, ayant tout type de peau au niveau des aisselles et ayant tendance à transpirer facilement.

Après une période de 10 jours comportant l'arrêt d'application de tout antiperspirant (wash-out) et utilisation exclusive d'un déodorant sans sels d'aluminium.
- Jour 1 : application au Centre - 5 pulvérisations sur l'aisselle à traiter selon un plan de randomisation, aucune application sur l'autre aisselle.
- A Jour 2 et Jour 3 : applications à domicile - 5 pulvérisations sur l'aisselle à traiter chaque jour selon un plan de randomisation,
- Jour 4 : application au Centre - 5 pulvérisations sur l'aisselle à traiter selon un plan de randomisation.

Critères d'évaluation :
- Evaluation par méthode gravimétrique de l'efficacité antiperspirante 24h suivant la dernière application, à l'issue d'une période de sudation en salle chauffée pendant 40 minutes précédée d'une période d'acclimatation de 20 minutes dans les mêmes conditions (recueil de sueur au cours de la période de stimulation thermique et enregistrement de la pesée du tampon après les fin de la période de stimulation thermique).
- Examen cutané des aisselles : L'éventuelle présence de rougeurs, sécheresse cutanée, petits boutons, sensations de tiraillements, sensations de picotements, démangeaisons a été notée par l'Investigateur ou par l'Assistante Clinique sous la responsabilité de l'Investigateur.

### RESULTATS DE L'ESSAI

Nombre de volontaires : 14 volontaires inclus ; 12 analysés (2 sorties d'essai). Evaluation par méthode gravimétrique de l'efficacité antiperspirante - Aisselles traitée et témoin au terme des 24 heures suivant la dernière application (n = 12)

| | **Poids de sueur collectée (en g) T24H** | | |
|---|---|---|---|
| | **Aisselle traitée** | **Aisselle témoin** | |
| **Moyenne** | **0,616** | **0,725** | |
| Ecart type | 0,593 | 0,610 | |
| Médiane | 0,430 | 0,465 | **Volontaires répondeurs (diminution ≥ 20% sur l'aisselle traitée)** |
| Minimum | 0,216 | 0,212 | |
| Maximum | 2,362 | 2,225 | |
| **% de variation sur la moyenne** | **-15,0%** | | **6/12** (50,0%) |

Conclusion : Dans les conditions expérimentales retenues, le produit SPRAY ANTI-PERSPIRANT selon l'invention, a présenté une efficacité antiperspirante 24 heures après la fin d'une série d'applications quotidiennes pendant 4 jours consécutifs au niveau des aisselles ; le critère d'efficacité retenu étant le suivant : obtenir une diminution d'au moins 20 % de la quantité de sueur recueillie sur l'aisselle traitée par rapport à l'aisselle témoin chez au moins 50 % des volontaires.

## Revendications

1. Utilisation cosmétique d'au moins un N-alkylamide comme anti transpirant, **caractérisée en ce que** ledit N-alkylamide est un sanshool.

2. Utilisation cosmétique d'au moins deux N-alkylamides différents comme anti transpirant, **caractérisée en ce que** lesdits deux N-alkylamides sont un sanshool et le spilanthol.

3. Utilisation cosmétique selon la revendication 1 ou 2, **caractérisée en ce que** le sanshool est choisi parmi: l'hydroxysanshool, l'α-sanshool, l'α-hydroxysanshool, le β-hydroxysanshool, le δ-sanshool, le γ-hydroxysanshool, le γ-hydroxyisosanshool, le y-dehydrosanshool et le γ-sanshool.

4. Utilisation cosmétique selon l'une des revendications 1 à 3, **caractérisée en ce que** la source de spilanthol est un extrait oléorésine *d'Acmella oleacerae* ou du spilanthol pur et **en ce que** la source de sanshools est un extrait CO₂ supercritique de Zanthoxylum *piperitum.*

5. Utilisation cosmétique selon l'une des revendications précédentes d'au moins un N-alkylamide en combinaison avec un activateur de pénétration, lequel est préférentiellement le nérolidol, dans une composition cosmétique.

6. Utilisation cosmétique selon l'une des revendications précédentes d'au moins un N-alkylamide dans une composition cosmétique, **caractérisée en ce que** la composition cosmétique est sous forme liquide, solide par exemple une crème, ou sous la forme d'un gel.

7. Procédé cosmétique pour réduire la transpiration, consistant à appliquer, par voie locale, une quantité efficace d'une composition cosmétique comprenant au moins un N-alkylamide en tant qu'agent actif, **caractérisé en ce que** ledit N-alkylamide est un sanshool.

8. Procédé cosmétique selon la revendication 7, **caractérisé en ce qu'**on applique une composition cosmétique comprenant au moins deux N-alkylamides. lesdits deux N-alkylamides étant un sanshool et le spilanthol.

## Patentansprüche

1. Kosmetische Verwendung von mindestens einem N-Alkylamid als Antitranspirant, **dadurch gekennzeichnet, dass** das genannte N-Alkylamid ein Sanshool ist.

2. Kosmetische Verwendung von mindestens zwei unterschiedlichen N-Alkylamiden als Antitranspirant, **dadurch gekennzeichnet, dass** die genannten zwei N-Alkylamide ein Sanshool und Spilanthol sind.

3. Kosmetische Verwendung nach Anspruch 1 oder 2, **dadurch gekennzeichnet, dass** das Sanshool ausgewählt ist aus: Hydroxysanshool, α-Sanshool, α-Hydroxysanshool, β-Hydroxysanshool, δ-Sanshool, γ-Hydroxysanshool, γ-Hydroxyisosanshool, γ-Dehydrosanshool und γ-Sanshool.

4. Kosmetische Verwendung nach einem der Ansprüche 1 bis 3, **dadurch gekennzeichnet, dass** die Quelle von Spilanthol ein Oleoresin-Extrakt von *Acmella oleacerae* oder rein Spilanthol ist und dass die Quelle von Sanshoolen ein überkritisches CO₂-Extrakt von *Zanthoxylum piperitum* ist.

5. Kosmetische Verwendung nach einem der vorhergehenden Ansprüche von mindestens einem N-Alkylamid in Kombination mit einem Penetrationsförderer, der vorzugsweise Nerolidol ist, in einer kosmetischen Zusammensetzung.

6. Kosmetische Verwendung nach einem der vorhergehenden Ansprüche von mindestens einem N-Alkylamid in einer kosmetischen Zusammensetzung in flüssiger Form, in fester Form, beispielsweise eine Creme, oder in der Form eines Gels ist.

7. Kosmetisches Verfahren zur Verringerung der Transpiration, das in dem lokalen Aufbringen einer wirksamen Menge einer kosmetischen Zusammensetzung besteht, die mindestens ein N-Alkylamid als Wirkstoff umfasst, **dadurch gekennzeichnet, dass** das genannte N-Alkylamid ein Sanshool ist.

8. Kosmetisches Verfahren nach Anspruch 7, **dadurch gekennzeichnet, dass** eine kosmetische Zusammensetzung aufgebracht wird, die mindestens zwei N-Alkylamide umfasst, wobei die genannten zwei N-Alkylamide ein Sanshool und Spilanthol sind.

## Claims

1. A cosmetic use of at least one N-alkylamide as anti-perspirant, **characterized in that** said N-alkylamide is a sanshool.

2. A cosmetic use of at least two different N-alkylamides as anti-perspirant, **characterized in that** said two N-alkylamides are a sanshool and the spilanthol.

3. The cosmetic use according to claim 1 or 2, **characterized in that** the sanshool is selected from: the hydroxysanshool, the α-sanshool, the α-hydroxysanshool, the β-hydroxysanshool, the δ-sanshool, the γ-hydroxysanshool, the γ-hydroxyisosanshool, the γ-dehydrosanshool and the γ-sanshool.

4. The cosmetic use according to any of claims 1 to 3, **characterized in that** the source of spilanthol is an oleoresin extract of *Acmella oleacerae* or pure spilanthol and **in that** the source of sanshools is a supercritical CO₂ extract of *Zanthoxylum piperitum.*

5. The cosmetic use according to any of the preceding claims, the at least one N-alkylamide being combined with a penetration activator, which is preferably the nerolidol, in a cosmetic composition.

6. The cosmetic use according to any of the preceding claims, the at least one N-alkylamide being included in a cosmetic composition in liquid form, in solid form, for example a cream, or in the form of a gel.

7. A cosmetic method for reducing perspiration, consisting in locally applying an efficient amount of a cosmetic composition comprising at least one N-alkylamide as an active ingredient, **characterized in that** said N-alkylamide is a sanshool.

8. The cosmetic method according to claim 7, **characterized in that** a cosmetic composition comprising at least two N-alkylamides is applied, said two N-alkylamides being a sanshool and the spilanthol.
